# EUROPÄISCHE PATENTSCHRIFT

(11) **EP 1 442 727 B1**
(45) Veröffentlichungstag und Bekanntmachung des Hinweises auf die Patenterteilung: **12.10.2016**
(21) Anmeldenummer: 04001933.3
(22) Anmeldetag: 29.01.2004
(51) Int. Cl.: A61F 2/34, A61F 2/32

(54) **Hüftgelenkprothese**
Hip joint prosthesis
Prothèse d'articulation de hanche

(30) Priorität: 31.01.2003 DE 10304102
(43) Veröffentlichungstag der Anmeldung: 04.08.2004
(73) Patentinhaber: CeramTec GmbH, 73207 Plochingen (DE); CeramConcept LLC, Newark, DE 19711 (US)
(72) Erfinder: Cruchet, Patrick, 72190 Coulaines (FR); Silberer, Paul, 68753 Waghäusel (DE); Bunz, Uwe, 72649 Wolfschlugen (DE); Dietrich, Martin, Dr., 23942 Pötenitz (DE); Masson, Bernard, 31320 Pechabou (FR)
(74) Vertreter: Uppena, Franz

(56) Entgegenhaltungen:
- EP-A- 0 821 922
- EP-A- 1 053 723
- WO-A-93/11722
- WO-A-96/04866
- WO-A-97/27827
- DE-A- 4 428 290
- FR-A- 2 662 930
- FR-A- 2 827 154
- FR-A- 2 827 504
- US-A- 5 681 872

## Beschreibung

Die Erfindung betrifft eine Hüftgelenkprothese zur Implantation bei Menschen und Tieren.

Bekannte Hüftgelenkprothesen bestehen aus einem Schaft, der in den Oberschenkelknochen implantiert wird und einem Kugelkopf, der auf dem Schaft z. B. durch eine konische Klemmung verankert ist. Der Kugelkopf rotiert hierbei in einer Pfanne. Die Pfanne kann direkt in das Hüftbecken implantiert werden oder aber in eine weitere äußere Schale oder in einen Kunststoffmantel eingesetzt und dann implantiert werden.

Bei den bekannten Hüftgelenkprothesen ist immer eine gewisse Luxationsneigung zu beobachten, d. h. dass der Kugelkopf bei gewissen Bewegungen aus der Pfanne herausrutscht. In der Fachliteratur finden sich einstellige Prozentangaben zur Luxationsneigung von Prothesensystemen wie Hüftgelenkprothesen.

Dieser Luxationsneigung könnte durch einen erhöhten Rand der Pfanne oder durch einen erhöhten Gleitpaarungsdurchmesser entgegengewirkt werden. Der Gleitpaarungsdurchmesser des Kugelkopfes bestimmt sich aus dem Durchmesser der äußeren Fläche des Kugelkopfes, die mit der Pfanne artikuliert.

Aus diesen konstruktiven Ausführungen resultieren jedoch verschiedene Nachteile. So schränkt z. B. ein erhöhter Rand an der Pfanne den Bewegungsraum des Kugelkopfes mit dem Schaft in der Pfanne stark ein. Der Einsatz größerer Gleitpaarungsdurchmesser, d. h. einen größeren Kugelkopf und eine größere Pfanne, ist limitiert durch Beschränkungen des zur Verfügung stehenden Einbauraumes.

Eine Hüftgelenkprothsre gemäß Oberbegriff des Anspruchs 1 wird im Dokument WO 97/27827 beschrieben.

Der Erfindung liegt die Aufgabe zu Grunde, bei Hüftgelenkprothesen die Luxationsneigung im Vergleich zum Stand der Technik zu verbessern.

Erfindungsgemäß wird diese Aufgabe gemäß den im kennzeichmenden Teil des Anspruchs 1 aufgeführten Merkmalen gelöst. Bevorzugt liegt das Durchmesserverhältnis der Gleitpaarung der Bipolarschale und des Kugelkopfes zwischen 1,05 und 5, vorzugsweise zwischen 1,2 und 2.

Vorteilhafterweise liegt der Gleitpaarungsdurchmesser der Bipolarschale zwischen 26 mm und 40 mm, bevorzugt bei 32 mm und der Gleitpaarungsdurchmesser des Kugelkopfes zwischen 14 mm und 32 mm, bevorzugt bei 22,2 mm.

Bei einer Hüftgelenkprothese mit einem keramischen Kugelkopf, einer keramischen Bipolarschale und einer keramischen Pfanne sind die tribologischen Bedingungen der keramischen Komponenten vorteilhafterweise durch eine Kombination folgender Merkmale definiert:
a) Die Härte der keramischen Komponenten ist größer als 1.000 HV (Vickers).
b) Die Oberflächenpolituren der artikulierenden Flächen der keramischen Komponenten haben eine Rauigkeit kleiner als 0,1 µm (Ra-Wert < 0,1 µm).
c) Der Benetzungswinkel der artikulierenden Flächen der keramischen Komponenten liegt zwischen 1° und 8° (gemessen in Ringerlösung).
d) Die Durchmesserdifferenz der Gleitpaarungsdurchmesser der artikulierenden Flächen der keramischen Komponenten liegt zwischen 1 und 200 µm, bevorzugt zwischen 20 und 120 µm

In bevorzugter Ausführungsform weisen die Drehzentren des Kugelkopfes zur Bipolarschale und der Bipolarschale zur Pfanne einen Versatz d auf und dieser Versatz d liegt zwischen 0,1 mm und 5 mm, vorzugsweise zwischen 1,5 und 2,5 mm.

Nachfolgend werden die Vorteile dieser Hüftgelenkprothese im Vergleich zum Stand der Technik beschrieben.
- Der Bewegungsspielraum (ROM = Range of motion) ist im Vergleich zu Systemen mit einem überhöhten Pfannenrand stark vergrößert.
- Die Luxationsneigung ist durch einen Verkeilungseffekt zwischen der Bipolarschale mit dem Sicherungsring und der Pfanne stark verringert.
- Die spezielle Kinematik und Tribologie führt zu einer von der einfachen Rotation differierenden Bewegung.

Der Bewegungsablauf spielt sich folgendermaßen ab:
Zuerst kommt es zu einer Bewegung zwischen Kugelkopf und Bipolarschale. Ist der Bewegungsspielraum dieser ersten Gleitfläche erschöpft, z. B. durch Anschlagen des Schaftes am Sicherungsring, kommt die zweite Gleitfläche zwischen Bipolarschale und Pfanne zum Einsatz; d. h. die weitere Bewegung findet nun an der äußeren Sphäre der Bipolarschale statt.

Durch die definierten tribologischen Eigenschaften und kinematischen Bedingungen gibt es keine reine Rotation um den Mittelpunkt der äußeren Sphäre der Bipolarschale, sondern es kommt zunächst zu einer Rotation der Bipolarschale um den Mittelpunkt des Kugelkopfes. Die Bipolarschale rotiert dabei aus der Pfanne heraus. Als Ergebnis dieser speziellen Koppelbewegung kommt es zu einer Verkeilung zwischen der Biopolarschale mit dem Sicherungsring und der Pfanne. Dadurch wird die Luxation deutlich erschwert, wie Messungen der Luxationskraft beweisen. Als Ergebnis ist die Luxationsneigung deutlich geringer.

### Materialien des Prothesensystems:

Die Prothese kann aus folgenden Materialien bestehen:
1. Prothesenschaft (Metall, Keramik, Kunststoff), vorzugsweise Metall
2. Kugelkopf (Keramik, Metall, Kunststoff) vorzugsweise Keramik
3. Bipolarschale (Metall, Keramik, Kunststoff), vorzugsweise Keramik
4. Sicherungsring (Metall, Keramik, Kunststoff), vorzugsweise Kunststoff
5. Pfanne oder Pfanneneinsatz (Metall, Keramik, Kunststoff), vorzugsweise Keramik.

Weitere Merkmale der Erfindung ergeben sich aus den Figuren, die nachfolgend beschrieben sind. Es zeigt:
- Fig. 1: das zum Kugelkopf gewandte Ende des Schaftes 1,
- Fig. 2: einen Kugelkopf 2,
- Fig. 3: einen Bipolarschale 3,
- Fig. 4: einen Sicherungsring 4 zum Einsatz in die Biopolarschale 3,
- Fig. 5: eine Pfanne 5,
- Fig. 6: eine Hüftgelenkprothese und
- Fig. 7: eine Hüftgelenkprothese mit Kennzeichnung des Versatzes d.

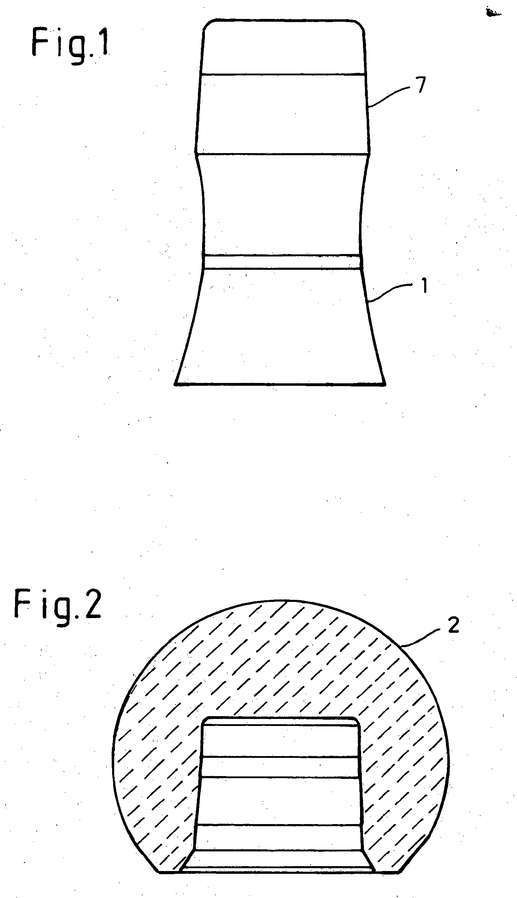

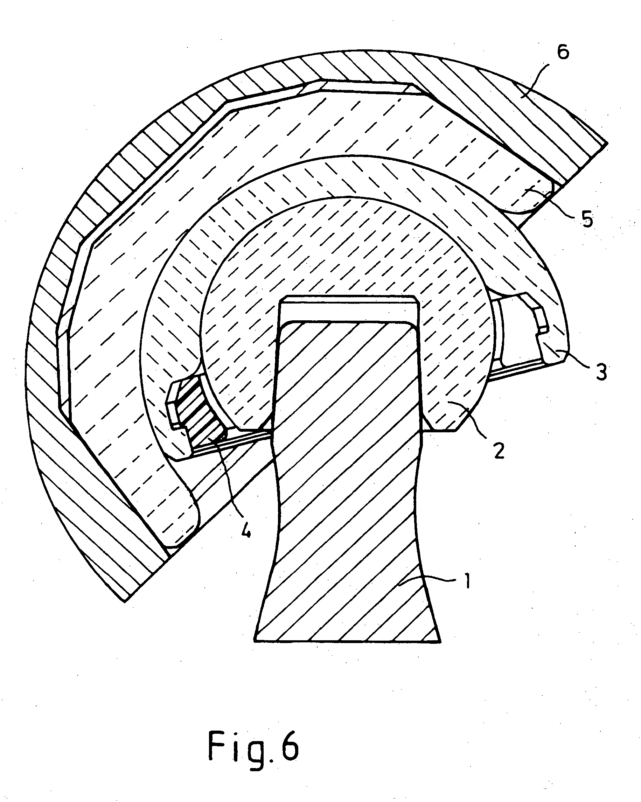

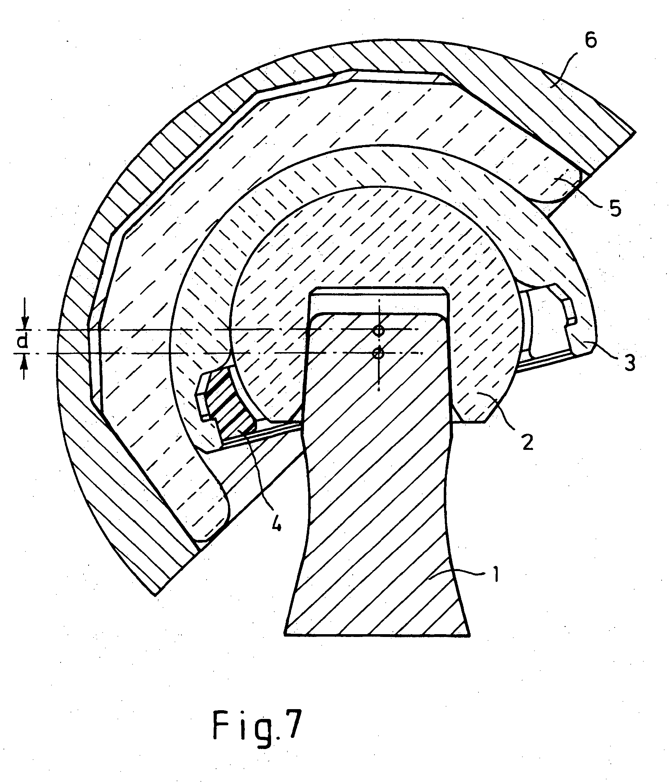

Die Figuren 1 bis 5 zeigen im Querschnitt die Einzelteile einer erfindungsgemäßen Ausführungsform einer Hüftgelenkprothese und die Figuren 6 und 7 einen Querschnitt einer kompletten Hüftgelenkprothese.

Fig. 1 zeigt den vorderen Teil eines Schaftes 1, der mit seinem nicht gezeigten Ende in den Oberschenkelknochen implantiert wird. Das gezeigte Ende des Schaftes 1 ist mit einer konischen Fläche 7 versehen. Diese konische Fläche 7 dient zur Befestigung eines Kugelkopfes 2, wie er in Fig. 2 gezeigt ist. Der Kugelkopf 2 weist eine Einbuchtung auf, die an ihrer Umfangsfläche ebenfalls mit einer konischen Fläche versehen ist, so dass der Kugelkopf 2 auf dem Schaft 1 befestigt werden kann.

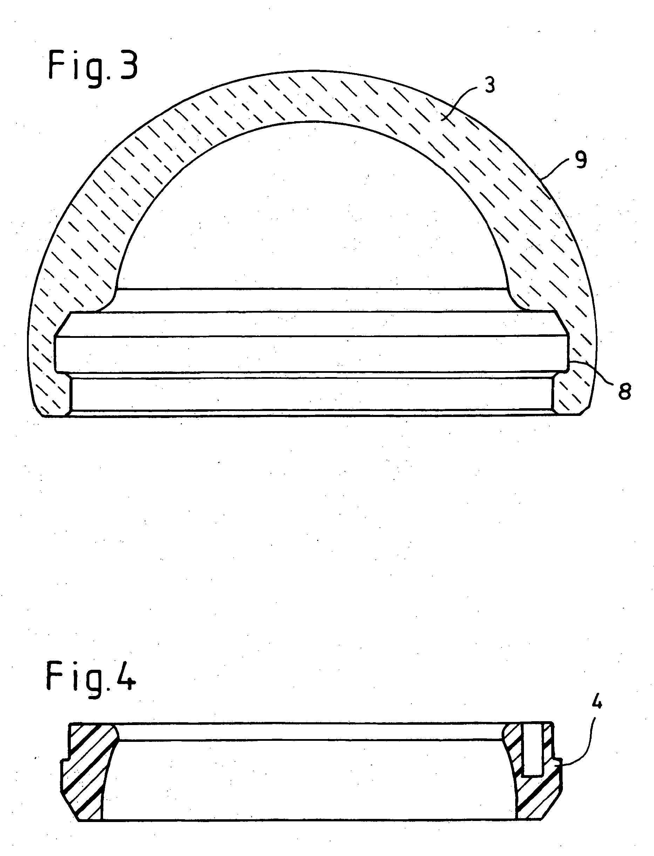

Fig. 3 zeigt eine Bipolarschale 3 mit einer sphärischen Außenfläche 9. Im Inneren der Bipolarschale 3 an ihrer zur Öffnung gewandten Seite ist eine Einbuchtung 8 angeordnet, in die ein Sicherungsring 4 (siehe Fig. 4) einsteckbar ist. Dieser Sicherungsring 4 dient zur Befestigung des Kugelkopfes 1 in der Bipolarschale 3.

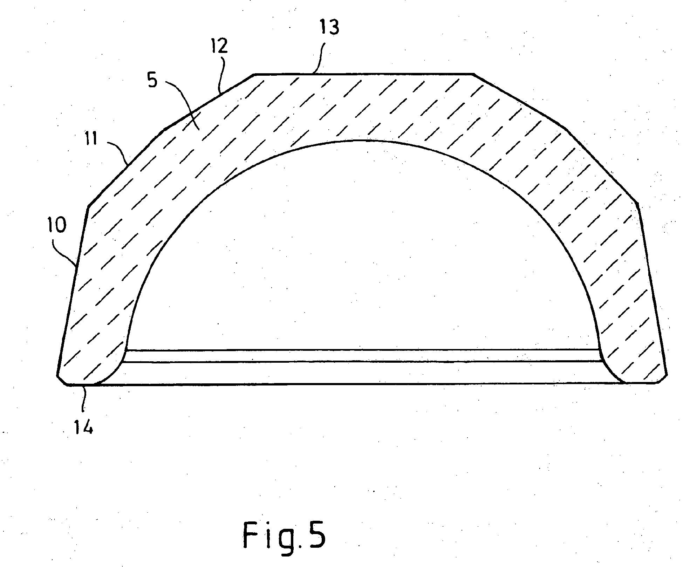

Fig. 5 zeigt eine Pfanne 5 mit einer sphärischen Einbuchtung, die zur Aufnahme der in Fig. 3 gezeigten Bipolarschale dient. An ihrer Außenseite ist die Pfanne 5 mit einer konischen Schräge 10 versehen, die über zwei Abflachungen 11, 12 in eine parallel zur Oberkante 14 verlaufende Abflachung 13 übergehen.

Die Figuren 6 und 7 zeigen den Zusammenbau der genannten Einzelkomponenten.

In dieser bevorzugten Ausführungsform besteht der Schaft 1 aus einem Metall (Titan), der Kugelkopf 2, die Bipolarschale 3 und die Pfanne 5 aus einer Keramik, die wie oben beschrieben speziell bearbeitet bzw. hergestellt sind. Die äußere Schale 6 in der die Pfanne 5 eingesetzt ist, besteht aus einem Metall. Optional kann diese Schale 6 auch entfallen, wenn die Pfanne 5 direkt in das Hüftbecken implantiert wird. Der mit dem Bezugszeichen 4 gekennzeichnete Sicherungsring ist aus Kunststoff hergestellt.

Fig. 7 zeigt den Versatz d der Drehzentren Kugelkopf 2 - Bipolarschale 3 und Bipolarschale 3 - Pfanne 5.

## Patentansprüche

1. Hüftgelenkprothese mit einem Schaft (1), der im Oberschenkelknochen implantierbar ist und mit einem keramischen Kugelkopf (2), der auf dem Schaft (1) z. B. durch eine konische Klemmung verankert ist, und mit einer Pfanne (5) in der der Kugelkopf (2) beweglich gelagert ist, wobei zwischen dem Kugelkopf (2) und der Pfanne (5) eine keramische Bipolarschale (3) angeordnet ist und der Kugelkopf (2) in der Bipolarschale (3) durch einen Sicherungsring (4) gehalten ist, wobei der Kugelkopf (2) in der Bipolarschale (3) und die Bipolarschale (3) in der Pfanne (5) rotiert und die Bipolarschale (3) im Querschnitt unterschiedliche Wandstärken aufweist, **dadurch gekennzeichnet**, das die Pfanne (5) eine keramische Pfanne (5) ist und die größte Wandstärke der keramischen Bipolarschale (3) im Bereich der Öffnung angeordnet ist und der Sicherungsring (4) am Rand der Bipolarschale (3) in diese eingesetzt ist.

2. Hüftgelenkprothese nach Anspruch 1, **dadurch gekennzeichnet, dass** das Durchmesserverhältnis der Gleitpaarung der Bipolarschale (3) und des Kugelkopfes (2) zwischen 1,05 und 5, vorzugsweise zwischen 1,2 und 2 liegt.

3. Hüftgelenkprothese nach Anspruch 2, **dadurch gekennzeichnet, dass** der Gleitpaarungsdurchmesser der Bipolarschale (3) zwischen 26 und 40 mm, bevorzugt bei 32 mm und der Gleitpaarungsdurchmesser des Kugelkopfes (2) zwischen 14 und 32 mm, bevorzugt bei 22,2 mm liegt.

4. Hüftgelenkprothese nach einem der Ansprüche 1 bis 3 mit einem keramischen Kugelkopf (2), einer keramischen Bipolarschale (3) und einer keramischen Pfanne (5), **dadurch gekennzeichnet, dass** die tribologischen Bedingungen der keramischen Komponenten (2, 3, 5) durch eine Kombination folgender Merkmale definiert sind:
a) Die Härte der keramischen Komponenten (2, 3, 5) ist größer als 1.000 HV (Vickers).
b) Die Oberflächenpolituren der artikulierenden Flächen der keramischen Komponenten (2, 3, 5) haben eine Rauigkeit kleiner als 0,1 µm (Ra-Wert < 0,1 m).
c) Der Benetzungswinkel der artikulierenden Flächen der keramischen Komponenten (2, 3, 5) liegt zwischen 1° und 8° (gemessen in Ringerlösung).
d) Die Durchmesserdifferenz der Gleitpaarungsdurchmesser der artikulierenden Flächen der keramischen Komponenten (2, 3, 5) liegt zwischen 1 und 200 µm, bevorzugt zwischen 20 und 120 µm.

5. Hüftgelenkprothese nach einem der Ansprüche 1 bis 4, **dadurch gekennzeichnet, dass** die Drehzentren des Kugelkopfes (2) zur Bipolarschale (3) und der Bipolarschale (3) zur Pfanne (5) einen Versatz (d) aufweisen und dieser Versatz (d) zwischen 0,1 mm und 5 mm, vorzugsweise zwischen 1,5 und 2,5 mm liegt.

## Claims

1. A hip joint prosthesis having a shaft (1), which can be implanted in the femur, and having a ceramic ball head (2), which is anchored on the shaft (1), for example by means of a conical clamp, and having a socket (5) in which the ball head (2) is movably mounted, wherein a ceramic bipolar shell (3) is arranged between the ball head (2) and the socket (5), and the ball head (2) is held in the bipolar shell (3) by means of a securing ring (4), wherein the ball head (2) rotates in the bipolar shell (3), and the bipolar shell (3) rotates in the socket (5), and the bipolar shell (3) has wall thicknesses that differ in cross section, **characterised in that** the socket (5) is a ceramic socket (5), and the greatest wall thickness of the ceramic bipolar shell (3) is arranged in the region of the opening, and the securing ring (4) is inserted into the bipolar shell (3) at the edge of the latter.

2. A hip joint prosthesis according to claim 1, **characterised in that** the ratio of the diameters of the sliding pairing of the bipolar shell (3) and of the ball head (2) lies between 1.05 and 5, preferably between 1.2 and 2.

3. A hip joint prosthesis according to claim 2, **characterised in that** the sliding-pairing diameter of the bipolar shell (3) lies between 26 and 40 mm, preferably at 32 mm, and the sliding-pairing diameter of the ball head (2) lies between 14 and 32 mm, preferably at 22.2 mm.

4. A hip joint prosthesis according to one of claims 1 to 3 having a ceramic ball head (2), a ceramic bipolar shell (3) and a ceramic socket (5), **characterised in that** the tribological conditions of the ceramic components (2, 3, 5) are defined by a combination of the following features:
a) the hardness of the ceramic components (2, 3, 5) is greater than 1,000 HV (Vickers);
b) the surface finishes of the articulating areas of the ceramic components (2, 3, 5) have a roughness that is less than 0.1 µm (Ra value < 0.1 m);
c) the wetting angle of contact of the articulating areas of the ceramic components (2, 3, 5) lies between 1° and 8° (measured in Ringer's solution);
d) the difference in diameter of the sliding-pairing diameters of the articulating areas of the ceramic components (2, 3, 5) lies between 1 and 200 µm, preferably between 20 and 120 µm.

5. A hip joint prosthesis according to one of claims 1 to 4, **characterised in that** the centres of rotation of the ball head (2) with respect to the bipolar shell (3) and of the bipolar shell (3) with respect to the socket (5) have an offset (d), and this offset (d) lies between 0.1 mm and 5 mm, preferably between 1.5 and 2.5 mm.

## Revendications

1. Prothèse de l'articulation de la hanche comprenant une tige (1), qui peut être implantée dans l'os fémoral, et comprenant une tête sphérique (2), qui est ancrée sur la tige (1), par exemple par un serrage conique, et comprenant un cotyle (5) dans lequel la tête sphérique (2) est montée de façon mobile, une cupule bipolaire (3) céramique étant disposée entre la tête sphérique (2) et le cotyle (5), et la tête sphérique (2) étant maintenue dans la cupule bipolaire (3) par une bague d'arrêt (4), la tête sphérique (2) tournant dans la cupule bipolaire (3) et la cupule bipolaire (3) tournant dans le cotyle (5), et la cupule bipolaire (3) présentant, en section transversale, des épaisseurs de paroi différentes, **caractérisée en ce que** le cotyle (5) est un cotyle (5) céramique et que l'épaisseur de paroi la plus importante de la cupule bipolaire (3) céramique est prévue dans la région de l'ouverture et que la bague d'arrêt (4) est insérée dans la cupule bipolaire (3), sur le bord de celle-ci.

2. Prothèse de l'articulation de la hanche selon la revendication 1, **caractérisée en ce que** le rapport de diamètre de l'appariement glissant de la cupule bipolaire (3) et de la tête sphérique (2) est compris entre 1,05 et 5, de préférence entre 1,2 et 2.

3. Prothèse de l'articulation de la hanche selon la revendication 2, **caractérisée en ce que** le diamètre d'appariement glissant de la cupule bipolaire (3) est compris entre 26 et 40 mm et est de préférence de 32 mm, et le diamètre d'appariement glissant de la tête sphérique (2) est compris entre 14 et 32 mm et est de préférence de 22,2 mm.

4. Prothèse de l'articulation de la hanche selon l'une des revendications 1 à 3, comprenant une tête sphérique (2) céramique, une cupule bipolaire (3) céramique et un cotyle (5) céramique, **caractérisée en ce que** les conditions tribologiques des composants (2, 3, 5) céramiques sont définies par une combinaison des caractéristiques suivantes :
a) La dureté des composants (2, 3, 5) céramiques est supérieure à 1 000 HV (Vickers).
b) Les polis de surface des surfaces d'articulation des composants (2, 3, 5) céramiques ont une rugosité inférieure à 0,1 µm (valeur Ra < 0,1 m).
c) L'angle de mouillage des surfaces d'articulation des composants (2, 3, 5) céramiques est compris entre 1° et 8° (mesuré dans une solution de Ringer).
d) La différence de diamètre des diamètres d'appariement glissant des surfaces d'articulation des composants (2, 3, 5) céramiques est comprise entre 1 et 200 µm, de préférence entre 20 et 120 µm.

5. Prothèse de l'articulation de la hanche selon l'une des revendications 1 à 4, **caractérisée en ce que** les centres de rotation de la tête sphérique (2) par rapport à la cupule bipolaire (3) et de la cupule bipolaire (3) par rapport au cotyle (5) présentent un décalage (d), et que ce décalage (d) est compris entre 0,1 mm et 5 mm, de préférence entre 1,5 et 2,5 mm.
